# FASCICULE DE BREVET EUROPEEN

(11) **EP 1 795 158 B1**
(45) Date de publication et mention de la délivrance du brevet: **03.09.2008**
(21) Numéro de dépôt: 05447272.5
(22) Date de dépôt: 06.12.2005
(51) Int. Cl.: A61F 7/12, A61F 6/06

(54) **Dispositif pour élever la température d'une cavité corporelle**
Vorrichtung zur Steigerung der Temperatur eines Körperhohlraums
Device for raising the temperature of a body cavity

(43) Date de publication de la demande: 13.06.2007
(73) Titulaire: Lardinois, Eric, 7100 Haine-Saint-Paul (BE); Maritan, Giorgio, 08810 Sant Pere de Ribas (ES); Petsch, Wolfgang, 1410 Waterloo (BE)
(72) Inventeur: Lardinois, Eric, 7100 Haine-Saint-Paul (BE); Maritan, Giorgio, 08810 Sant Pere de Ribas (ES); Petsch, Wolfgang, 1410 Waterloo (BE)
(74) Mandataire: Cauchie, Daniel

(56) Documents cités:
- EP-A- 0 672 400
- CH-A- 337 612
- GB-A- 384 179
- GB-A- 1 143 149
- US-A- 2 192 768

## Description

La présente invention se rapporte, d'une manière générale, à un dispositif capable d'élever la température d'une cavité corporelle.

Plus précisément, l'invention se rapporte à un dispositif capable d'élever la température d'une cavité corporelle notamment la cavité rectale et plus particulièrement la cavité vaginale, du genre comprenant un corps creux de forme oblongue, pour insertion dans la cavité corporelle, comportant une paroi latérale, une paroi transversale d'extrémité supérieure et une paroi transversale d'extrémité inférieure, ce corps creux étant muni intérieurement d'une source de chaleur et extérieurement d'un moyen de retrait de la cavité corporelle.

Il existe à ce jour de nombreuses méthodes contraceptives féminines parmi lesquelles on peut citer des méthodes basées sur l'interprétation de la température intravaginale, des méthodes hormonales ou encore des méthodes mécaniques incluant la pose de dispositifs tels que diaphragme, stérilet ou condom féminin.

Toutes ces méthodes et moyens sont destinés à être utilisés préalablement à un coït en vue d'empêcher une conception.

Toutefois, lorsqu'aucun moyen contraceptif n'a été utilisé dans ces conditions ou s'est révélé déficient alors qu'une conception non désirée est prévisible, une contraception d'urgence ou postcoïtale peut être envisagée. Ce type de méthode, destinée à empêcher une fécondation en vue de prévenir une grossesse non planifiée, représente le stade ultime de l'ensemble des variétés de contraception avant un éventuel avortement.

On connaît à ce jour, deux types de méthodes contraceptives postcoïtales, l'une chimique faisant appel soit uniquement à un progestatif tel qu'une progestine par exemple le lévonorgestrel soit à une association orale oestro-progestative comprenant par exemple la progestérone et une progestine comme le lévonorgestrel, et l'autre mécanique basée sur la mise en place d'un dispositif intra-utérin genre stérilet.

Toutefois, ces méthodes ne sont pas dénuées d'effets secondaires indésirables.

A ce titre, on signalera que l'administration d'hormones est susceptible de provoquer l'apparition de différents effets secondaires tels que nausées, vomissements, saignements vaginaux irréguliers, douleurs abdominales, vertiges, asthénie, mastodynies ou encore céphalées, alors qu'un dispositif intra-utérin devrait être déconseillé aux sujets à risques élevés d'infection sexuellement transmissibles. Il est connu, en effet, que l'insertion d'un tel dispositif peut conduire à une infection pelvienne capable de provoquer une infertilité en cas d'absence de traitement ou pis, de constituer un terrain propice à une invasion du virus de l'immunodéficience humaine (VIH) en cas d'exposition à celui-ci.

En raison notamment de ces inconvénients et effets secondaires indésirables, de telles méthodes nécessitent l'aide du corps médical.

Le développement d'une méthode contraceptive postcoïtale qui soit à la fois non toxique, non hormonale, principalement dépourvue d'effets secondaires, faisant appel à des moyens réutilisables et faciles à mettre en place, de préférence sans l'aide du corps médical, reste par conséquent d'un intérêt primordial.

On sait que les spermatozoïdes présentent une morphologie vulnérable, instable et sensible à un excès de chaleur même minime.

Ainsi, une augmentation de 0,5°C par rapport à la température corporelle normale est déjà suffisante pour désorganiser leur motilité.

D'autre part, à une température de l'ordre de 42°C à 44°C, les spermatozoïdes subissent un éclatement et une scission entre la tête et la queue empêchant leur motilité alors qu'à environ 45°C, la tête subit un éclatement irréversible les rendant totalement inefficaces.

Une méthode contraceptive masculine basée sur un excès calorifique a été décrite dans la demande de brevet DE 4 041 254 mettant en oeuvre un dispositif sous forme d'une pochette chauffée à une température de 39°C à 40°C c'est-à-dire légèrement supérieure à la température corporelle. Selon cette demande de brevet, la pochette, positionnée autour des testicules, en totalité, perturbe la production et la motilité des spermatozoïdes, en raison de l'augmentation de chaleur, produisant ainsi une infertilité passagère chez l'homme.

Toutefois, après un coït, les spermatozoïdes ayant été soumis à un tel traitement se trouveront dans un milieu de confinement totalement différent de celui qu'ils occupaient dans les testicules puisqu'ils subiront l'influence de divers facteurs et mécanismes inhérents à la spère génitale féminine.

Il est connu, en effet, que les spermatozoïdes déposés dans la cavité vaginale peuvent être guidés jusqu'au site de fertilisation grâce à un mécanisme thermique mettant en jeu leur attirance vers la chaleur plus importante émise par le site de fécondation de l'ovule.

En d'autres termes, les spermatozoïdes, après coït, sont susceptibles d'entreprendre dans le tractus génital, une migration par signaux thermiques et, durant cette migration, d'acquérir sous l'effet de différents facteurs contrôlés par la physiologie féminine, une maturité nécessaire à une fécondation.

Pour ces raisons, le comportement postcoïtal de spermatozoïdes issus de testicules dont seule l'enveloppe extérieure a été en contact avec un dispositif porté à une température aussi faible que 39°C à 40°C, puis mis en présence de la sphère vaginale à température corporelle normale, ne peut apparaître que totalement imprévisible. Aussi, l'efficacité d'un tel effet contraceptif peut-elle être valablement mise en doute. En conséquence, la méthode décrite dans la susdite demande de brevet, basée sur une légère augmentation de la température du milieu de confinement testiculaire des spermatozoïdes par rapport à la température corporelle normale, n'apparaît pas comme une méthode contraceptive fiable.

Or, il a été trouvé, dans le cadre de l'invention, qu'un apport de chaleur suffisant pour provoquer une augmentation de la température intravaginale au-delà de la température naturelle, essentiellement une augmentation de la température jusqu'au moins 42°C à 45°C, est capable, *in situ,* après un coït et avant fécondation, de provoquer une désorganisation de la motilité et une altération directe et suffisante de la morphologie des spermatozoïdes pour inhiber leur migration jusqu'au site de fécondation et, en conséquence, l'acquisition durant cette migration de la maturité nécessaire à une fécondation, induisant ainsi un effet contraceptif, c'est-à-dire une prévention de fécondation.

Cette altération des spermatozoïdes conduisant à leur destruction peut être produite et stimulée *in situ,* par différents facteurs en cascade, dans la totalité du tractus génital femelle, en particulier dans la sphère génitale dans son ensemble c'est-à-dire y compris l'entrée du vagin.

En outre, une telle augmentation de la température à l'intérieur du vagin jusqu'à et y compris le cervix s'est révélée capable, après un coït et/ou après fécondation, de constituer un préliminaire à une interruption précoce de grossesse, ou avortement spontané, par suite d'une création de conditions défavorables au maintien de l'ovule fécondé dans l'utérus.

Au surplus, une augmentation de la température intravaginale du même ordre, avant coït, s'est montrée apte à créer des conditions favorables à une contraception suite à la survenue de processus physiologiques complexes capables de défavoriser toute étape d'évolution des spermatozoïdes dans l'ensemble du tractus génital ainsi que l'implantation de l'ovule dans la paroi déciduale, provoquant ainsi son expulsion spontanée de l'utérus.

En particulier, un apport supplémentaire de chaleur avant un coït induit déjà au niveau du tractus génital, en particulier de la sphère vaginale, des conditions telles qu'en cas de nécessité d'augmentation de la température intravaginale après le coït, le temps efficace et disponible pour provoquer une telle augmentation de température après coït sera moins important que si aucune augmentation de température n'avait été apportée avant le coït.

Ces différentes observations permettent, en conséquence, de proposer une méthode pour générer des conditions défavorables à l'installation d'une grossesse chez la femme nécessitant ces conditions, méthode selon laquelle on assure un apport de chaleur, à l'ensemble de la cavité vaginale y compris son entrée, pour provoquer une augmentation de la température intravaginale au-delà de la température corporelle jusqu'à au moins 42°C à 45°C de manière à induire *in situ* :
a) après un coït et avant fécondation, une désorganisation de la motilité et une altération directe et suffisante de la morphologie des spermatozoïdes pour inhiber leur objectif de migration jusqu'au site de fécondation et leur possibilité d'acquérir une maturité nécessaire à la fécondation, et ainsi produire un effet contraceptif,
b) après un coït et après fécondation, des conditions défavorables au maintien de l'ovule fécondé dans l'utérus, éventuellement en présence d'une ou de plusieurs prostaglandines naturelles et/ou d'un ou de plusieurs dérivés de prostaglandines, de façon à constituer un préliminaire à une interruption précoce de grossesse, et même de produire un effet abortif,
c) avant un coït, des conditions favorables à une contraception par suite de processus physiologiques capables de perturber les facteurs qui déterminent la maturité des spermatozoïdes et de défavoriser l'implantation de l'ovule dans la paroi déciduale provoquant ainsi son expulsion de l'utérus.

De nos jours, le syndrome d'immunodéficience acquise (SIDA) constitue un véritable défi en raison du fléau qu'il représente et qu'il s'agit de contrôler et d'enrayer par tous moyens possibles à chacune des étapes du développement de l'infection, notamment dès l'introduction du virus dans l'hôte.

On sait, par exemple, qu'après un contact sexuel à risques se traduisant par une exposition possible au VIH, un traitement prophylactique peut être envisagé. Toutefois, celui-ci doit être entrepris impérativement après le contact sexuel ou dans les 24 heures de manière à bénéficier des meilleurs potentialités cependant sans garantie d'un évitement de l'infection virale. Après 72 heures, le traitement préventif s'avérerait sans espoir de pouvoir contrôler la progression du virus.

Une inoculation du VIH, immédiatement après exposition sexuelle à celui-ci, peut cependant s'avérer très faible, ce qui pourrait augmenter l'efficacité, durant cette période cruciale, d'un traitement prophylactique simple ou multiple.

Toutefois, la réplication virale est capable de « mémoriser » les mécanismes qui, à travers toute l'évolution du virus, conduisent à des modifications de celui-ci en réponse à certains facteurs extérieurs. En conséquence, ce phénomène de mémorisation pourrait représenter une échappatoire aux médicaments anti-rétroviraux et aux futurs vaccins.

Par contre, lorsque ce facteur extérieur est représenté par une augmentation de la température corporelle au-delà de 37°C, par exemple une température de l'ordre de 43°C à 55°C, le VIH est incapable de mettre en place des mécanismes d'adaptation mais au contraire subit des modifications irréversibles qui le fragilisent et le rendent vulnérable au système immunitaire. En finalité, le VIH dénonce sa présence au système immunitaire grâce à une quantité de chaleur suffisante.

La méthode selon l'invention est tout à fait appropriée pour permettre, grâce à la quantité suffisante de chaleur qu'elle apporte, de provoquer une dénonciation du VIH au système immunitaire au contraire de la méthode selon la demande de brevet allemand DE 4041254 qui met en oeuvre une quantité de chaleur totalement insuffisante pour atteindre ce but.

Comme dans le cas d'autres infections virales, l'introduction du VIH dans l'hôte initie des réponses immédiates du système immunitaire dans son ensemble qui, dans la majorité des cas, contrôle partiellement la réplication virale. Des éléments de l'immunité innée sont activés lesquels stimulent le système immunitaire d'adaptation pour contrôler ensemble la propagation du VIH.

Des approches pour induire d'abord l'immunité innée apparaissent évidentes et constituent une priorité première des soins de santé. En d'autres termes, agir avant la survenue de l'infection représente une opportunité non négligeable d'empêcher un pré cancer, un cancer non invasif ou toute prolifération virale dans l'ensemble de la sphère génitale et notamment dans la cavité vaginale.

Or, il a également été trouvé qu'un apport de chaleur suffisant pour provoquer une augmentation de la température intravaginale au-delà de la température corporelle, essentiellement une augmentation de la température jusqu'au moins 55°C est capable, *in situ,* avant et après un coït, de déclencher un ensemble de phénomènes appelés « réponse au choc thermique » caractérisés entre autres, par la synthèse de protéines de même nom.

Ces protéines de choc thermique et/ou de stress thermique (HSP) surexprimées jouent alors un rôle supérieur de protection (« chaperons ») vis-à-vis des autres constituants polypeptidiques des cellules, en ce qu'elles sont capables de stabiliser les protéines en configuration anormale et de jouer un rôle dans le repliement et le dépliement des protéines en général. On peut citer à cet effet la protéine de choc thermique HSP70 d'importance primordiale ainsi que la protéine HSP27 dont l'expression chez la femme est élevée dans l'endomètre utérin, le vagin et le cervix.

Comme les protéines de choc thermique sont capables d'agir sur le système immunitaire ainsi que de le contrôler, une production accrue de celles-ci peut constituer une aide précieuse pour stimuler les mécanismes de défense de l'organisme et le système immunitaire en général. A ce titre, la protéine HSP70 de même que les autres membres de la famille des HSP, s'est révélée capable de fonctionner comme un facteur inné anti-VIH afin de limiter son cycle de transmission et de réplication.

En conséquence, l'apport de chaleur dont il est question ci-dessus sera apte à déclencher et/ou à activer le système immunitaire génital en particulier le système immunitaire génital inné et ce, par l'intermédiaire de la protéine de choc et/ou de stress, de manière à générer une réponse favorable dans différents problèmes féminins incluant des irritations ou encore dans le processus de prévention et même de traitement de certains cancers (pré cancers et cancers non invasifs) tels que cancer du vagin, du cervix, de l'utérus ou des ovaires ou encore de certaines maladies infectieuses ou virales, en particulier des infections sexuellement transmissibles notamment le SIDA.

Ces différentes constatations permettent, en conséquence, de proposer une méthode capable d'activer le système immunitaire génital de manière à générer une réponse favorable dans le processus de prévention et de traitement de cancers, essentiellement pré cancers et cancers non invasifs et de maladies infectieuses ou virales notamment des maladies sexuellement transmissibles, en particulier le SIDA, chez la femme nécessitant une telle réponse favorable, méthode selon laquelle on assure un apport de chaleur à l'ensemble de la cavité vaginale y compris son entrée, pour provoquer une augmentation de la température corporelle jusqu'à au moins 55°C de manière à induire in situ un déclenchement de protéines de choc thermique et/ou de stress thermique capables de stimuler ledit système immunitaire génital.

Des dispositifs capables d'assurer une augmentation de la température d'une cavité corporelle en particulier la température rectale ou vaginale sont déjà connus. Toutefois, leur usage est limité à un effet thérapeutique.

Ainsi, on a décrit dans le brevet GB 384 179 un dispositif pour élever ou abaisser la température d'une cavité corporelle comprenant un support métallique d'un élément tubulaire qui traverse ce support de part en part et dont la portion supérieure est destinée à être introduite dans la cavité corporelle. Cet élément tubulaire est fermé à l'extrémité de sa portion supérieure située du côté de la paroi supérieure du support et relié, par l'autre extrémité située du côté de la paroi inférieure de ce support, à un robinet à trois voies lui-même en liaison avec une source extérieure de chaleur ou de froid. Par ailleurs, une enveloppe amovible peut être posée par dessus la portion supérieure de l'élément tubulaire et solidarisée à la partie supérieure du support par l'intermédiaire d'un anneau à visser et d'un joint d'étanchéité.

De même, on a rapporté dans le brevet US 2 192 768 un dispositif destiné à appliquer de la chaleur aux organes génito-urinaires. Ce dispositif comprend un corps cylindrique divisé en deux chambres séparées, l'une d'entrée, l'autre de sortie. Une double boucle creuse, destinée à être introduite dans la cavité corporelle, relie entre elles les extrémités supérieures de ces chambres. En outre, ce dispositif comprend deux conduits, l'un reliant l'extrémité inférieure de la chambre d'entrée à une source d'eau chaude pour introduction dans les boucles, l'autre reliant l'extrémité inférieure de la chambre de sortie pour l'expulsion de l'eau hors des boucles.

Au surplus, on a décrit dans le brevet US 3 170 465 un dispositif permettant d'apporter une certaine quantité de chaleur à différentes parties du corps notamment à la cavité rectale ou vaginale. Ce dispositif peut être considéré comme autonome car capable de produire lui-même et *in situ* cette chaleur à partir d'une réaction exothermique produite par des composés chimiques, initialement séparés par une cloison, puis mis en contact, par exemple, après rupture de cette cloison.

Aucune précision n'est apportée quant à la nature de ces produits chimiques ou au type de réaction produite, ce qui laisse supposer que celle-ci n'est pas réversible et que, par conséquent, le dispositif en question n'est pas réutilisable. D'autre part, ce dispositif n'est pas mentionné comme capable d'induire un effet contraceptif. Par ailleurs, le patient destiné à recevoir ce dispositif doit garder l'immobilité lors de sa mise en place qui doit être effectuée par du personnel qualifié.

D'autre part, on a rapporté dans la demande de brevet WO 2004/009004 un dispositif pour le traitement thermique thérapeutique du vagin féminin au départ d'un corps de forme cylindrique dont les bords sont arrondis et émoussés, ce corps cylindrique comportant un cordon de retrait fixé à l'une de ses extrémités. Ce dispositif, en l'occurrence le corps cylindrique, contient un milieu accumulateur de chaleur, celle-ci provenant d'un bain extérieur d'eau chaude ou d'un chauffage par micro-ondes.

Toutefois, ce dispositif, s'il est réutilisable, n'apparaît pas autonome car incapable d'auto-produire la chaleur désirée *in situ.* Par conséquent, un tel dispositif nécessite un apport de chaleur extérieur immédiatement avant usage, ce qui peut engendrer certaines contraintes.

La présente invention a pour but de proposer un dispositif du type indiqué précédemment, capable de produire de la chaleur dans une cavité corporelle, en particulier la cavité vaginale, de façon à en augmenter la température et de pallier les inconvénients ou déficiences évoqués précédemment, ce dispositif étant notamment utilisable et éventuellement réutilisable en toute sécurité et pouvant être posé directement par l'utilisateur ou l'utilisatrice sans l'intervention nécessaire du corps médical.

Pour atteindre ce but, le dispositif du type décrit précédemment est **caractérisé en ce que :**
- la paroi transversale d'extrémité inférieure du corps creux est reliée au moyen de retrait par l'intermédiaire d'un moyen apte à échanger de la chaleur entre d'une part ledit corps creux et d'autre part soit ledit moyen de retrait, soit au moins un anneau enserrant ledit moyen échangeur de chaleur, soit ledit moyen de retrait et au moins un anneau enserrant ledit moyen échangeur de chaleur et la paroi transversale d'extrémité supérieure du corps creux comporte éventuellement un moyen apte à échanger de la chaleur entre ledit corps creux et au moins un anneau enserrant ledit moyen échangeur de chaleur,
   ou
- la paroi transversale d'extrémité inférieure du corps creux est reliée au moyen de retrait et la paroi transversale d'extrémité supérieure du corps creux comporte un moyen apte à échanger de la chaleur entre ledit corps creux et au moins un anneau enserrant ledit moyen échangeur de chaleur,
en sorte que la source de chaleur génère une quantité de chaleur capable d'augmenter la température de la cavité corporelle totale jusqu'à et y compris son entrée, en particulier la cavité vaginale totale.

Selon une caractéristique particulière et avantageuse de l'invention, le corps creux est relié au moyen de retrait par l'intermédiaire d'un moyen apte à échanger de la chaleur entre ledit corps creux et ledit moyen de retrait, un anneau insérant éventuellement ledit moyen échangeur de chaleur.

Selon une autre caractéristique de l'invention, la source de chaleur, génère une quantité de chaleur intravaginale capable de mettre en application la méthode décrite précédemment notamment d'empêcher la migration des spermatozoïdes entre autres dans le cervix et les zones supérieures de fertilisation telles que les trompes de Falope ainsi que l'acquisition d'une maturité nécessaire à la fécondation et, en conséquence, de produire un effet contraceptif.

Selon une caractéristique supplémentaire de l'invention, la source de chaleur génère une quantité de chaleur intravaginale capable de mettre en application la méthode décrite précédemment notamment de déclencher et/ou stimuler le système immunitaire génital femelle. Ainsi, cette quantité de chaleur supplémentaire s'avère capable, entre autres, de déclencher la production de protéines de choc thermique et/ou de stress thermique qui jouent un rôle entre l'immunité innée et le système immunitaire d'adaptation par l'intermédiaire de cytokines et d'interféron.

Le corps creux du dispositif selon l'invention, de forme oblongue appropriée pour une insertion intravaginale ou rectale, comprend une extrémité supérieure d'insertion et, à l'opposé, une extrémité inférieure de retrait munie d'un moyen échangeur de chaleur.

La forme, la taille, la stabilité de ce corps creux peut être symétrique ou asymétrique par rapport à l'axe central longitudinal de celui-ci.

Généralement, cette forme sera sensiblement cylindrique, de surface extérieure non rugueuse essentiellement adoucie et dépourvue d'angles vifs, ceux-ci étant avantageusement arrondis ou émoussés de manière à éviter blessures et traumatismes par exemple une perforation du canal vaginal lors de l'introduction du corps creux, un syndrome inflammatoire provoqué par un corps étranger ou une contamination bactérienne.

Toutefois, la forme initiale de ce corps creux peut être modifiée notamment par rapport à son axe transversal de façon à épouser intimement la forme de la cavité vaginale ou rectale et en particulier de la cavité vaginale supérieure située au niveau du cervix. A titre d'exemple, la paroi longitudinale cylindrique du corps creux peut subir sur sa hauteur totale ou partielle une ou plusieurs enflures ou un ou plusieurs renflements. Ces modifications de forme sont disposées de manière régulière ou irrégulière sur le pourtour total du corps creux. A titre d'exemple, la région du pourtour du corps creux située sensiblement à mi-chemin entre les extrémités d'insertion et de retrait peut comporter un renflement dont le périmètre équivaut à 95 % à 75 % du périmètre de l'extrémité d'insertion et/ou de l'extrémité de retrait.

Par ailleurs, les parois transversales d'extrémité d'insertion et/ou de retrait peuvent comporter, indépendamment l'une de l'autre, au moins une enflure vers l'extérieur ou au moins un renflement vers l'intérieur. Ces modifications peuvent concerner la surface de ces parois d'extrémité, en totalité ou partiellement.

Les longueur et largeur hors tout de ce corps creux sont avantageusement déterminées de manière appropriée en tenant compte des différents coefficients de dilatation des matériaux constitutifs de l'enveloppe qui le délimite. Ainsi, la longueur du corps creux en question, c'est-à-dire la longueur hors tout séparant les extrémités des deux parois transversales est généralement égale ou inférieure à 150 mm, de préférence 65 mm et la largeur hors tout de ce même corps creux est habituellement égale ou inférieure à 50 mm, de préférence 23 mm.

Le corps creux du dispositif selon l'invention comporte une enveloppe extérieure formée d'un film de matériau approprié ou de deux ou plusieurs films de matériaux appropriés différents de coefficient de dilatation différents à la chaleur. Ces films combinés peuvent être associés en étant fixés ensemble et/ou scellés de manière à donner lieu à un profil souhaitable de dilatation de l'ensemble de telle sorte qu'à chaque augmentation de température, à l'intérieur du corps creux, la dilatation du ou des films situés le plus à l'extérieur soit plus lente que la dilatation du ou des films situés le plus à l'intérieur de ce corps creux.

Habituellement, cette enveloppe extérieure, d'une épaisseur d'environ 0,01 à 0,8 mm de préférence d'environ 0,02 mm à environ 0,5 mm, est réalisée en un ou plusieurs matériaux stables et solides mais encore souples, flexibles, élastiques et de préférence capables de « s'auto-cicatriser ». A ce titre, le matériau décrit dans la demande de brevet WO2005/046540 et commercialisé sous la marque FFLEXX^{®} (Groupe Wellcare Products) constitue un matériau de choix lequel permettra notamment un scellement particulièrement efficace.

Les matériaux utilisables pour l'élaboration de l'enveloppe du corps creux seront non toxiques et biocompatibles et, en outre, pourront résister, en toute sécurité, aussi bien à toute variation de chaleur qu'aux différents composants entrant dans la solution sursaturée pour éviter notamment leur diffusion au travers de cette enveloppe. De manière à assurer la sécurité voulue, ces matériaux devront être capables par ailleurs de résister à différentes sollicitations physiques journalières de pression interne ou externe notamment lors du retrait manuel du dispositif représenté essentiellement par le corps creux. Dans ce contexte, le niveau de sécurité doit être d'environ 200 kg.

Ainsi l'enveloppe de ce corps creux peut être formée d'un film de matériau polymérique ou encore d'une autre matière souple conventionnelle apte à contenir des liquides chauffés par exemple le caoutchouc ou le silicone.

Des matériaux polymériques utilisables, peuvent être sélectionnés parmi le polyéthylène, le polypropylène, le polyamide, le polyester, le chlorure de polyvinyle, le chlorure de polyvinylidène, le polystyrène, le polyuréthanne, un copolymère d'acétate d'éthylène-vinyle saponifié ou non. En outre, ces matériaux peuvent être associés à des tissus tissés ou non, tissus adaptés pour entrer en contact avec des liquides chauffés.

Selon les nécessités, l'enveloppe du corps creux est réalisée en un matériau, de structure spongieuse ou poreuse, apte à être recouvert et/ou imprégné d'agents biocompatibles ou encore contenir de tels agents choisis notamment parmi des agents spermiostatiques, spermicides, anti-infectieux, virucides, bactéricides, des prostaglandines naturelles et des dérivés de prostaglandines ou, en général, choisis parmi tout agent médicamenteux ou non, utilisable notamment par voie rectale ou vaginale ou toute composition contenant de tels agents.

En outre, selon une caractéristique particulière et avantageuse, le corps creux est entouré d'une gaine ou enveloppe amovible réalisée en un matériau capable d'être recouvert et/ou imprégné d'un ou de plusieurs agents biocompatibles, médicamenteux ou non, choisis parmi les agents et compositions cités précédemment.

Plus particulièrement, le corps creux en question peut servir de support à une telle enveloppe utile elle-même comme support d'un médicament administrable par voie rectale et/ou vaginale. Il s'agit, en particulier de médicaments dont la résorption par la muqueuse rectale ou vaginale est favorisée lors d'une augmentation de la température de ce site d'absorption.

Selon les nécessités, l'enveloppe en question montée amovible sur le corps creux et véhiculant un agent médicamenteux ou non, choisi parmi la famille évoquée précédemment, pourra être facilement ôtée et remplacée par une enveloppe analogue véhiculant un autre agent de cette famille.

Le moyen échangeur de chaleur, situé au niveau de la paroi transversale inférieure peut, habituellement comprendre une proéminence inférieure provenant de celle-ci surmontée d'une protubérance provenant de cette proéminence ou rapportée sur celle-ci, protubérance dans laquelle le moyen de retrait peut coulisser librement ou être partiellement intégré.

Lorsque le moyen de retrait est apte à coulisser librement, cette protubérance peut se présenter en une partie mais avantageusement en plusieurs parties dans le but essentiel d'accroître la surface externe de l'échangeur et par conséquent les échanges calorifiques.

A titre d'exemple, cet échangeur de chaleur peut comprendre une protubérance dont une portion de sa paroi est commune avec une portion ou la totalité de la proéminence inférieure de ce corps creux.

En conséquence, selon un mode de réalisation particulier, la protubérance dans laquelle coulisse le moyen de retrait peut être surmontée d'une coiffe amovible, généralement en un matériau bon conducteur calorifique, qui peut être en contact uniquement avec cette protubérance ou également avec la proéminence inférieure du corps creux.

Par ailleurs, lorsque le moyen de retrait est partiellement intégré dans la protubérance faisant partie du moyen échangeur de chaleur, une portion maximale de la surface de ce moyen de retrait est en contact avec cette protubérance elle-même en contact avec une portion maximale de la proéminence inférieure.

Selon une forme de réalisation particulière, le corps creux comporte, en sus du moyen échangeur de chaleur au niveau de sa paroi transversale inférieure, un moyen échangeur de chaleur supplémentaire situé au niveau de sa paroi transversale supérieure, ce moyen supplémentaire comprenant une proéminence supérieure provenant de cette paroi ou rapportée sur celle-ci.

Toutefois, selon une autre forme de réalisation de l'invention, le corps creux est dépourvu d'échangeur de chaleur à sa paroi transversale inférieure mais est muni d'un tel échangeur de chaleur au niveau de sa paroi transversale supérieure.

Le moyen de retrait de la capsule est généralement constitué d'un moyen manuel tel qu'un cordon de retrait habituellement en matière souple de façon à ne pas interférer dans les activités normales de la vie journalière. Il est formé, en général, d'un brin unique, creux ou non, se terminant par une boucle distale pour assurer un retrait aisé du corps creux ou, de préférence, d'un brin, creux ou non, en forme d'anneau, l'un ou l'autre de ces brins étant attaché directement ou indirectement au corps creux.

Selon un autre mode de réalisation, le moyen de retrait est formé d'une languette de retrait comportant éventuellement un bourrelet périphérique creux ou non.

Ce moyen de retrait constitue, en outre, un élément de sécurité supplémentaire pour protéger automatiquement la zone située entre les organes génitaux femelles externes et la cavité vaginale. D'autre part, ce moyen de retrait étant visible extérieurement au niveau des organes génitaux, un contrôle périodique de sa présence ou de sa position correcte peut être opéré par l'utilisatrice.

Selon une autre caractéristique de l'invention, ce moyen de retrait peut être imprégné et/ou recouvert d'un ou de plusieurs agents biocompatibles, médicamenteux ou non, choisis parmi des agents spermiostatiques, spermicides, anti-infectieux, virucides et bactéricides formulés par exemple sous forme d'une crème.

Lorsqu'il est creux, le moyen de retrait renferme avantageusement un ou plusieurs agents de transfert calorifique de sorte que le taux de libération des agents biocompatibles en question peut être contrôlé lorsque ce moyen de retrait est chauffé efficacement. Ces agents, qui peuvent se présenter sous forme solide, liquide ou gazeuse, sont sélectionnés parmi des matières capables d'assurer une bonne conduction et/ou convection de la chaleur recueillie à partir de l'échangeur calorifique. Lorsqu'il est sous forme solide, l'agent de transfert calorifique peut comprendre un ou plusieurs fils métalliques souples et bons conducteurs de chaleur. Ces fils occupent avantageusement la totalité du creux à l'intérieur du moyen de retrait et peuvent être considérés, par conséquent, comme noyés dans la masse de ce moyen de retrait. Lorsque cet agent de transfert calorifique est un liquide, celui-ci sera choisi en sorte que sa température d'ébullition soit supérieure à la température d'ébullition du liquide dans lequel le dispositif selon l'invention sera réchauffé après utilisation.

De tels agents liquides, certains d'entre eux ayant été décrits à titre d'exemples dans le brevet US 5 417 276, seront thermiquement stables, sûrs, non toxiques et présenteront une viscosité suffisante pour éviter une diffusion au travers du moyen de retrait. Par ailleurs, lorsque cet agent de transfert calorifique est sous forme liquide, celui-ci sera préférentiellement non volatile pour des raisons de sécurité.

En outre, selon une caractéristique supplémentaire de l'invention, le moyen échangeur de chaleur situé au niveau de la paroi transversale inférieure et, lorsqu'il est présent, le moyen échangeur de chaleur au niveau de la paroi transversale supérieure est préférentiellement enserré par un anneau juxtaposé à cette paroi, en particulier un anneau circulaire.

Toutefois, selon un mode de réalisation particulier, seule la paroi transversale supérieure comporte, juxtaposé, un anneau enserrant l'échangeur de chaleur, l'échangeur de chaleur au niveau de la paroi transversale inférieure étant dépourvu d'un tel anneau.

Avantageusement, cet anneau est configuré de façon à ne pas empêcher un écoulement des fluides vaginaux qui participent notamment au transfert calorifique et au transfert de facteurs immunitaires provenant de sécrétions vaginales et cervicales.

Au contraire, un tel anneau tend davantage à favoriser un drainage de ces fluides sans un quelconque blocage de ceux-ci le long de la muqueuse vaginale. Ces fluides, lorsqu'ils sont porteurs d'un ou de plusieurs médicaments, permettront une meilleure répartition et une meilleure absorption de ceux-ci par la muqueuse vaginale ou cervicale. En outre, ces fluides assureront une intégrité tissulaire de façon à maintenir un environnement vaginal sain en dépit de variations cycliques. Pour ces raisons, un anneau de forme torique est particulièrement préféré.

Après la pose du dispositif selon l'invention, cet anneau, lorsqu'il est juxtaposé à l'extrémité de retrait de ce dispositif, se situera à proximité de l'orifice vaginal alors que l'anneau éventuellement juxtaposé à l'extrémité d'insertion sera localisé à proximité immédiate du cervix.

Cet anneau, en relation avec le ou les moyens échangeurs de chaleur, peut être recouvert et/ou être imprégné d'agents biocompatibles, médicamenteux ou non, ou encore contenir de tels agents choisis parmi des agents spermiostatiques, spermicides, anti-infectieux, virucides, bactéricides, des prostaglandines naturelles et des dérivés de prostaglandines. Lorsqu'il est imprégné de ces agents biocompatibles ou lorsqu'il les contient, l'anneau laissera diffuser ces composés grâce à sa structure respectivement spongieuse ou poreuse prévue à cet effet. Toutefois, ces prostaglandines et/ou dérivés de prostaglandines, étant donné leur effet abortif, seront davantage localisés au niveau de l'extrémité d'insertion du corps creux.

Alternativement, les extrémités de retrait et d'insertion peuvent être munies chacune d'un anneau torique tel que précédemment recouvert et/ou imprégné d'agents biocompatibles comme décrit précédemment.

Selon une caractéristique supplémentaire de l'invention, la source de chaleur correspond à un système auto-producteur de chaleur *in situ* capable d'élever la témpérature d'une cavité corporelle jusqu'au moins 55°C. Ainsi, selon un mode de réalisation particulièrement avantageux, ce système auto-producteur de chaleur *in situ* comprend une solution saline sursaturée et surrefroidie et un moyen de déclenchement d'une cristallisation exothermique du ou des sels de la solution. En particulier, ce système auto-producteur de chaleur comprend une solution saline sursaturée et surrefroidie formée d'une solution d'au moins un composé inorganique dans un milieu aqueux contenant un alcool, ainsi qu'un déclencheur apte à provoquer le déclenchement de la cristallisation souhaitée et la production concomitante de chaleur.

Le composé inorganique est généralement choisi à partir d'un acétate métallique de préférence un acétate de métal alcalin ou alcalino-terreux comme l'acétate de sodium et d'un nitrate métallique de préférence un nitrate de métal alcalin ou alcalino-terreux comme le nitrate de calcium alors que l'alcool est habituellement un polyalcool choisi parmi l'éthylèneglycol et le glycérol.

En outre, la solution en question peut éventuellement contenir de manière avantageuse un agent capable notamment d'accroître la durée de validité du dispositif selon l'invention tel qu'une amine par exemple l'aniline ou tout autre agent capable de maintenir l'équilibre de la solution sursaturée recristallisable en particulier des nano particules.

Cette solution recristallisable peut être obtenue selon des méthodes conventionnelles comprenant la préparation d'une solution aqueuse du composé inorganique par exemple dans l'eau distillée, l'ajout à cette solution d'un alcool avec agitation de l'ensemble puis ajout d'aniline également avec agitation du milieu résultant.

Le moyen de déclenchement, quant à lui, comprend un système de déclenchement ou déclencheur ainsi qu'éventuellement un ou plusieurs moyens de support et/ou de protection. Le système de déclenchement, formé d'au moins un diaphragme de déclenchement, est capable de présenter deux configurations extrêmes entre lesquelles il peut être fléchi par un déplacement instantané, provoquant une onde d'oscillation à l'intérieur du système auto-producteur de chaleur fermé et le démarrage concomitant d'une cristallisation exothermique progressive.

En raison d'une fonction d'oscillation à fréquence de base unique, ce diaphragme de déclenchement est apte, par ailleurs, à reprendre automatiquement sa configuration initiale après une action quelconque ayant provoqué sa déformation de façon, si nécessaire, à redémarrer un nouveau cycle suite à une nouvelle sollicitation de déformation.

Cet « effet mémoire », présenté par certains matériaux métalliques, peut être provoqué lorsque ceux-ci sont soumis à un choc thermique notamment le choc thermique généré par la réaction exothermique de cristallisation du ou des sels et/ou par le processus de redissolution des sels en question lors d'un apport extérieur de chaleur notamment au moyen d'eau bouillante.

Différents types de diaphragme de déclenchement à déformation brusque, capables d'être utilisés dans le dispositif selon l'invention, sont connus ayant été décrits dans l'état de la technique. A ce titre, on peut citer le brevet EP 0 464 092 qui rapporte un diaphragme de déclenchement, se présentant sous forme d'un ruban ou lamelle flexible et mince, avantageusement muni de rainures ou perforé de fines fentes espacées, pour maximaliser la réaction d'oscillation ondulatoire. Ce diaphragme, dont la bordure périphérique est avantageusement de forme circulaire ou elliptique, présente une portion centrale bombée et habituellement de même forme que celle du bord extérieur du diaphragme en question.

En outre, ce diaphragme, dont la surface extérieure est convexe, c'est-à-dire la surface sur laquelle une sollicitation de pression doit être exercée, peut comporter une ou plusieurs rainures généralement en forme de V dont la pointe est tournée vers la surface extérieure. La déformation du diaphragme, sous l'action d'une pression, provoque une ouverture plus importante des rainures puis, lors du retour de celui-ci à sa position initiale, un rétrécissement de cette ouverture et une compression résultante de la solution saline provoquant, en conséquence, une initiation de sa cristallisation.

Dans le cadre de la présente invention, on propose toutefois un moyen de déclenchement comprenant différentes caractéristiques qui, prises dans leur ensemble, rendent celui-ci particulièrement avantageux. Ce moyen de déclenchement adapté aux dimensions restreintes du dispositif de l'invention allie à la fois performance, résistance, longévité et facilité d'utilisation.

Ainsi, selon une première caractéristique avantageuse de ce moyen de déclenchement, l'épaisseur du diaphragme varie depuis son centre jusqu'à son pourtour extérieur en fonction des différentes zones de tension créées lors d'une pression exercée axialement sur ce diaphragme pour en provoquer sa déformation. Ces différentes épaisseurs auront pour avantage de répartir, de façon homogène, dans ce diaphragme, les tensions internes ainsi générées. Par conséquent, les zones de surtension pourront être réduites au maximum contribuant ainsi à une plus grande fiabilité dans la résistance mécanique du diaphragme en question. Par ailleurs, l'épaisseur de ce dernier peut également varier selon la viscosité du milieu de recristallisation dans lequel il séjourne.

Cette configuration du diaphragme ainsi décrit permettant d'en accroître la résistance face aux sollicitations de tension auxquelles il est soumis, influencera, par conséquent, de manière nettement favorable son efficacité et sa durée de validité.

Aussi, un dispositif selon l'invention comprenant un tel diaphragme de déclenchement allié à une solution saline sursaturée contenant une amine peut être utilisé des centaines de fois de manière fiable et durant des mois sinon des années.

Selon une autre caractéristique particulière de l'invention, la portion bombée du diaphragme de déclenchement comporte trois fentes d'égale longueur qui s'étendent symétriquement, à partir de son centre, par conséquent dans des directions séparées de 120°, et qui se terminent chacune par un orifice, en particulier circulaire ou elliptique. Généralement ces fentes qui peuvent être réalisées par laser, présentent une largeur de 0,1 à 0,5 mm par exemple 0,3 mm. Toutefois, le diaphragme de déclenchement qui initie l'onde d'oscillation, constitue un système physique qui peut vibrer indépendamment des fentes en question lorsqu'il est soumis à une force extérieure.

Les orifices, de préférence semblables, présentent une largeur plus importante que les fentes et ont notamment pour fonction d'absorber les tensions développées lors de l'exercice d'une pression sur ce diaphragme pour le déformer. Dans cette réalisation, les bords des fentes, sont arrondis ou biseautés pour éviter toute friction opérationnelle et même tout contact entre eux aussi bien en équilibre stable, c'est-à-dire au repos du diaphragme, qu'en une position de déformation c'est-à-dire en cours de déformation ou en équilibre instable, à savoir en fin de course de déformation.

Selon une autre caractéristique de l'invention, les fentes sont configurées de façon à passer, lors d'une déformation du diaphragme sous l'effet d'une pression, par une position de rapprochement maximal capable de créer une compression de la solution saline entre lesdites fentes avec un dégagement suffisant de chaleur pour initier une cristallisation exothermique du ou des sels de la solution.

En outre, selon une caractéristique particulièrement avantageuse de l'invention, le diaphragme de déclenchement comprend une portion bombée prenant la forme générale d'un triangle équilatéral à côtés légèrement recourbés vers l'intérieur et à sommets arrondis. Pour des raisons de commodité, ce triangle sera dénommé par la suite « triangle curviligne ».

Un tel triangle bombé présente notamment l'avantage de permettre une excellente répartition des différentes forces développées lors d'une pression exercée sur cette portion bombée à partir de sa face convexe.

D'autre part, selon une autre caractéristique avantageuse de l'invention, la portion bombée du diaphragme de déclenchement comprend trois rainures semblables situées entre les orifices et sur une circonférence dont le centre équivaut à celui de cette portion bombée.

De même, selon une caractéristique supplémentaire de l'invention, le diaphragme de déclenchement comprend, à l'extérieur de la portion bombée, un bourrelet non périphérique qui s'étend transversalement à la fois du côté concave et du côté convexe de ce diaphragme.

Ce bourrelet présente notamment l'avantage de renforcer l'équilibre et la solidité du diaphragme et d'absorber l'énergie et les tensions générées lors d'une pression sur celui-ci.

Selon une autre caractéristique de l'invention, le diaphragme de déclenchement est fabriqué à partir d'un alliage à mémoire de forme (« shape memory alloy ») essentiellement à partir d'acier inoxydable, d'un alliage Ni/Ti ou d'une combinaison des deux, dont la structure hétérogène est constituée de cristaux particulièrement fins.

Ainsi, ce système de déclenchement est habituellement réalisé en une matière non corrosive flexible de préférence en acier inoxydable, éventuellement combiné à un alliage Ni/Ti, et présentant des caractéristiques d'eutectique. Cet acier est avantageusement traité lors de sa fusion de manière à donner naissance à une masse hétérogène en fusion formée de cristaux extrêmement fins, puis refroidi à vitesse particulièrement lente de façon à maintenir cette structure hétérogène constituée de cristaux extrêmement fins.

Par conséquent, le diaphragme de déclenchement est fabriqué à partir d'un matériau dont la structure est identique à celle qu'il possède lors de sa fusion, ce qui lui confère une durée de vie très importante et une sécurité fiable.

Selon une autre caractéristique particulièrement avantageuse de l'invention, le moyen de déclenchement comprend un diaphragme de déclenchement, au moins trois pieds supports ainsi que deux éléments de guidage et/ou de protection, l'un et l'autre solidarisés au diaphragme de déclenchement.

Ces éléments support et de guidage, de forme et de dimensions sensiblement identiques à celles du diaphragme, sont fixés à celui-ci par l'intermédiaire d'au moins trois pieds supports et sont situés de part et d'autre de ce diaphragme, l'élément support et/ou de protection du côté concave, l'élément de guidage et/ou de protection du côté convexe.

Etant donné les dimensions restreintes du système de déclenchement, l'élément support ainsi que l'élément de guidage contribuent notamment au repérage de ce système dans le corps creux par exemple lorsque ce repérage doit s'effectuer uniquement au toucher.

Selon des mises en oeuvre différentes, le système de déclenchement peut être simple ou multiple c'est-à-dire qu'il peut comprendre une seule lamelle ou au contraire plusieurs lamelles, par exemple deux lamelles alignées axialement.

Quelle que soit la configuration adoptée pour le système de déclenchement selon l'invention et en raison notamment de ses dimensions restreintes, son actionnement nécessitera l'utilisation uniquement de deux doigts d'une même main d'un utilisateur ou d'une utilisatrice.

Le déclencheur, qui est inséré dans le corps creux, peut flotter librement dans la solution recristallisable. Pour cette raison, ce déclencheur, en particulier le diaphragme de déclenchement, est exempt de bords acérés de manière à éviter des perforations ou déchirures de l'enveloppe du corps creux. Cependant, de manière à augmenter la sécurité nécessaire, ce diaphragme peut être muni d'un bourrelet annulaire courant le long de sa périphérie. D'autre part, le déclencheur peut être positionné de manière fixe à tout endroit de l'intérieur de ce corps creux. Avantageusement, ce déclencheur peut être inséré à l'opposé de l'extrémité d'insertion dans le canal vaginal c'est-à-dire dans la région de l'extrémité de retrait, de manière à le protéger de tout déclenchement imprévisible d'une cristallisation saline exothermique due à une force extérieure non contrôlée. En particulier, le déclencheur se situera le long de l'axe de symétrie longitudinal de ce corps creux.

L'invention sera mieux comprise et d'autres buts, caractéristiques et avantages de celle-ci apparaîtront plus clairement au cours de la description explicative qui va suivre faite en référence aux dessins annexés donnés uniquement à titre d'exemples illustrant différents modes de réalisation de l'invention et dans lesquels :
- la figure 1 est une vue éclatée d'un dispositif, selon l'invention, pour élever la température de la cavité vaginale,
- la figure 2 est une vue en plan d'un diaphragme de déclenchement d'une cristallisation,
- la figure 3 est une vue en perspective du diaphragme à la figure 2, montrant les lignes de force,
- la figure 4 est une vue en coupe verticale du diaphragme à la figure 2,
- la figure 5 est une vue en perspective d'un moyen de déclenchement d'une cristallisation,
- la figure 6 est une vue en coupe d'un autre mode de réalisation d'un dispositif selon l'invention,
- la figure 7 est une vue en perspective du dispositif à la figure 6.
- la figure 8 est une vue en coupe horizontale d'un autre mode de réalisation du dispositif à la figure 6.
- La figure 9 est une vue en plan coupe verticale du mode de réalisation du dispositif à la figure 8.

### EXEMPLE 1

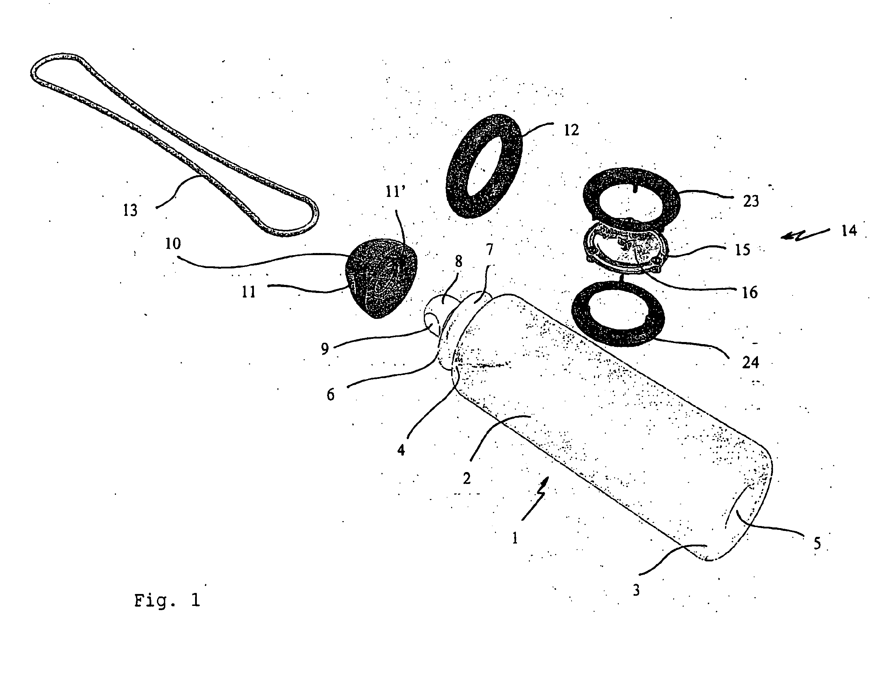

Tel que représenté à la figure 1, le dispositif selon l'invention comprend un corps creux 1 allongé et d'une longueur d'environ 65 mm. Ce corps creux cylindrique, d'environ 19 mm de diamètre, est formé d'une paroi 2, d'environ 0,1 mm d'épaisseur dont les bords circulaires sont fortement arrondis.

Ce corps creux possède deux extrémités fermées chacune par une paroi transversale, l'une 3 en tant qu'extrémité d'insertion dans le canal vaginal, l'autre 4 en tant qu'extrémité de retrait de celui-ci. L'extrémité 3 présente par ailleurs un renflement 5 également sensiblement circulaire.

En outre, la paroi 2 s'étend en une proéminence 6 circulaire et de préférence creuse munie d'une gorge radiale 7, cette proéminence se terminant par une protubérance 8 en forme d'oeillet d'attache traversé de part en part par un orifice 9. L'ensemble formé par la paroi 2, la proéminence 6 à gorge et l'oeillet 8 est fabriqué en un matériau stable et non toxique généralement d'un seul tenant. Avantageusement, cet ensemble qui peut provenir de moulage, par exemple à partir d'un polyéthylène, est recouvert d'une fine pellicule d'un matériau thermiquement stable, non toxique et conducteur de chaleur, notamment un matériau métallique.

Cet oeillet est surmonté d'un capuchon 10 comportant deux orifices 11 et 11' qui, lorsque ce capuchon est posé sur l'oeillet 8, se trouvent l'un et l'autre en regard de l'orifice 9. Ce capuchon, également recouvert d'un matériau conducteur de chaleur, complète le système échangeur de chaleur déjà constitué par la proéminence 6 et l'oeillet 8.

L'ensemble ainsi formé comporte, en outre extérieurement un anneau torique 12, préférentiellement de structure spongieuse entourant la proéminence 6 et épousant étroitement la forme de la gorge 7 et d'autre part un cordon de retrait 13 également en polyéthylène souple prenant la forme d'un anneau passant par les orifices 11, 9 et 11' et apte à glisser librement dans ceux-ci. L'anneau torique 12 et le cordon 13 sont avantageusement recouverts, si nécessaire, d'un composé spermiostatique et/ou spermicide et si nécessaire d'un médicament tel qu'un anti-infectieux et/ou un antiviral et/ou une ou plusieurs prostaglandines ou dérivés de celles-ci.

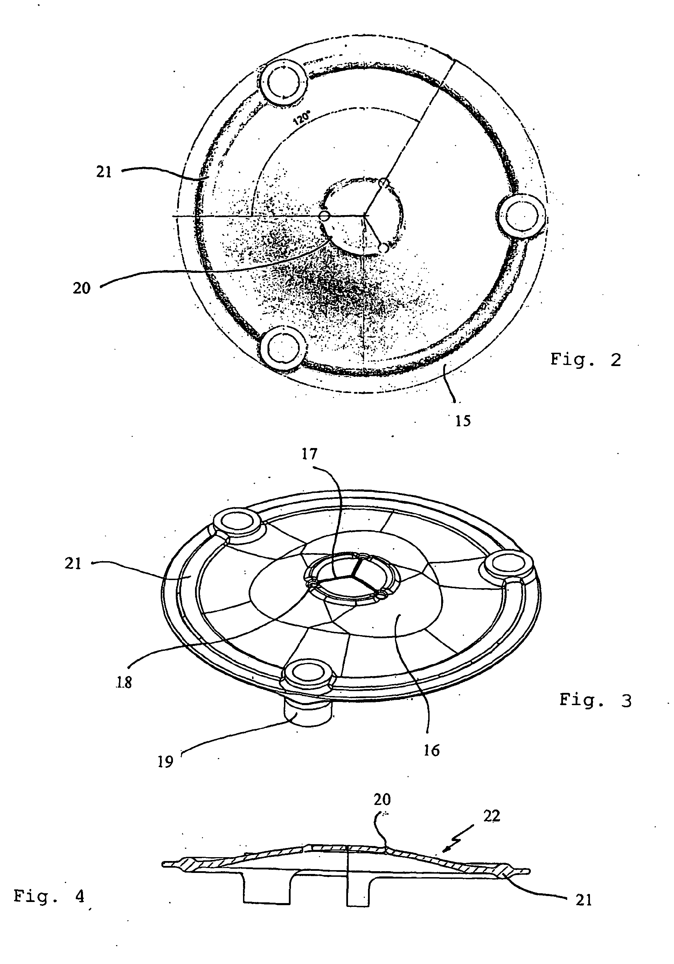

La figure 1 montre également les différentes parties d'un déclencheur 14. Tel qu'il apparaît aux figures 2 et 3, ce déclencheur est formé, pour l'essentiel, d'un diaphragme 15 ou fine lamelle circulaire, en acier inoxydable comprenant un alliage Ni/Ti, d'ou émerge une portion 16 bombée prenant la forme générale d'un triangle équilatéral curviligne. Ce triangle, centré sur cette lamelle est matérialisé sous forme d'un trait plein à la figure 3.

Cette figure 3 montre également, s'éloignant du centre de la lamelle 15 circulaire, trois fentes 17 identiques, séparées l'une de l'autre de 120° et se terminant chacune par un orifice 18 circulaire. Avantageusement les bords de ces fentes sont parallèles et biseautés et les orifices sont identiques.

D'autre part, ce diaphragme est muni de trois pieds supports 19. Ceux-ci partiellement évidés à partir de leurs extrémités et de forme globalement cylindrique s'étendent de part et d'autre du diaphragme en question. On remarque également que les centres de ces pieds supports, situés chacun sur une circonférence concentrique au diaphragme et à l'intersection avec une médiane d'un côté du triangle en question, sont séparés l'un de l'autre de 120°.

La figure 2 montre également trois rainures 20 prenant la forme de trois portions identiques d'une même circonférence concentrique au diaphragme. Ces rainures en arc de cercle, disposées de manière symétrique entre elles, définissent trois arcs de cercle identiques formant des zones de séparation dont le centre de chacun d'eux se trouve positionné sur un rayon passant par le sommet du triangle curviligne. Ainsi, ces centres sont également séparés l'un de l'autre de 120°.

On remarque également à la figure 4 que l'ouverture des rainures se situe du côté concave du diaphragme et aux figures 2 et 3 que les centres des pieds circulaires se trouvent positionnés au niveau d'un bourrelet annulaire 21 de renforcement du système de déclenchement.

Ce bourrelet, concentrique au diaphragme, s'étend symétriquement de part et d'autre de celui-ci. Ainsi, une force résultant d'une pression quelconque exercée au centre de la surface convexe d'un tel diaphragme de configuration totalement symétrique se répartit en six composantes d'égale intensité selon six axes séparés chacun de 60°, la force résultant au niveau du bourrelet s'avérant nulle.

D'autre part, on peut observer également à la figure 4 que l'épaisseur du diaphragme varie depuis la rainure 20 jusqu'au bourrelet 21, celle-ci étant la plus faible au niveau de 22.

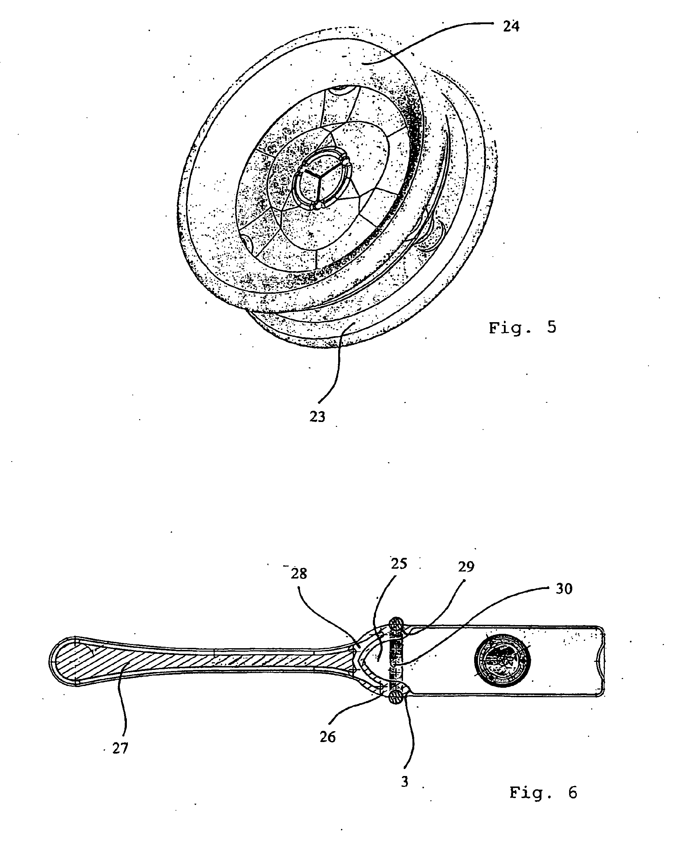

Le diaphragme de déclenchement ainsi décrit est complété par deux rondelles 23 et 24, visibles aux figures 1 et 5, l'une et l'autre formant support et/ou bouclier de guidage et/ou de protection. Ces rondelles sont constituées d'une paroi circulaire en polyéthylène s'évasant vers l'extérieur leur conférant une forme de type tulipe. Chacune d'elles est munie à sa face inférieure, globalement concave, de trois ergots (non visibles) aptes à s'engager dans les évidements des pieds supports du diaphragme en sorte que celui-ci soit maintenu à proximité immédiate des rondelles 23 et 24.

A la lecture de ce qui précède, on comprend aisément qu'après un coït, lorsqu'un effet contraceptif est encore souhaité avant fécondation, il suffit d'introduire le corps creux dans la cavité vaginale par son extrémité d'insertion 3 et de le pousser, manuellement ou à l'aide d'un applicateur, jusqu'au voisinage du cervix ou à l'intérieur de celui-ci. Au préalable, au aura initié la cristallisation des sels dissous dans le milieu renfermé dans ce corps creux de manière à provoquer la production d'un dégagement de chaleur. A cet effet, on exerce une pression soudaine sur le système de déclenchement 15 au niveau du centre de la portion 16 suivie d'un relâchement de celle-ci et ce, par l'intermédiaire de deux doigts l'un positionné sur la surface convexe du diaphragme l'autre sous la rondelle 23.

Sous l'effet de cette pression, le diaphragme bombé se déforme brusquement. Au cours de cette déformation, les bords des fentes 17, éloignés l'un de l'autre au départ, se rapprochent de plus en plus pour passer par une position de proximité maximale puis s'éloignent l'un de l'autre jusqu'à fin de course de déformation. Lors du relâchement de cette pression manuelle, ce diaphragme retrouve immédiatement et automatiquement sa forme primitive. Toutefois, au moment de leur rapprochement maximal, une surpression est engendrée entre les bords des fentes 17 avec dégagement de chaleur suffisant pour provoquer une amorce de cristallisation de la solution saline comprimée. Cette cristallisation se poursuit alors produisant un dégagement de chaleur capable d'atteindre 38°C à 55°C à l'intérieur de la cavité vaginale. La recherche manuelle du système de déclenchement immergé dans la solution saline du corps creux est en outre facilitée par les rondelles 23 et 24 dont la configuration en tulipe permet de guider deux doigts de l'utilisatrice jusqu'au centre de ce diaphragme.

D'autre part, l'introduction correcte du corps creux dans la cavité vaginale peut être aisément contrôlée lorsque l'anneau torique 12 se situe au niveau de la protubérance 6 en ce que cet anneau devra se situer dans le vagin au niveau de son entrée, le cordon 13 étant situé également partiellement dans le vagin.

Lors du retrait du corps creux, dès que la réaction exothermique est terminée ou pour toute autre raison, il suffit d'exercer une force de traction sur le cordon 13 pour libérer le corps creux de la cavité vaginale.

Si nécessaire, le dispositif de l'invention pourra être réutilisé après avoir ramené, à l'état liquide, la solution cristallisée et ce, par chauffage de cette solution cristallisée par exemple dans l'eau chaude.

### EXEMPLE 2

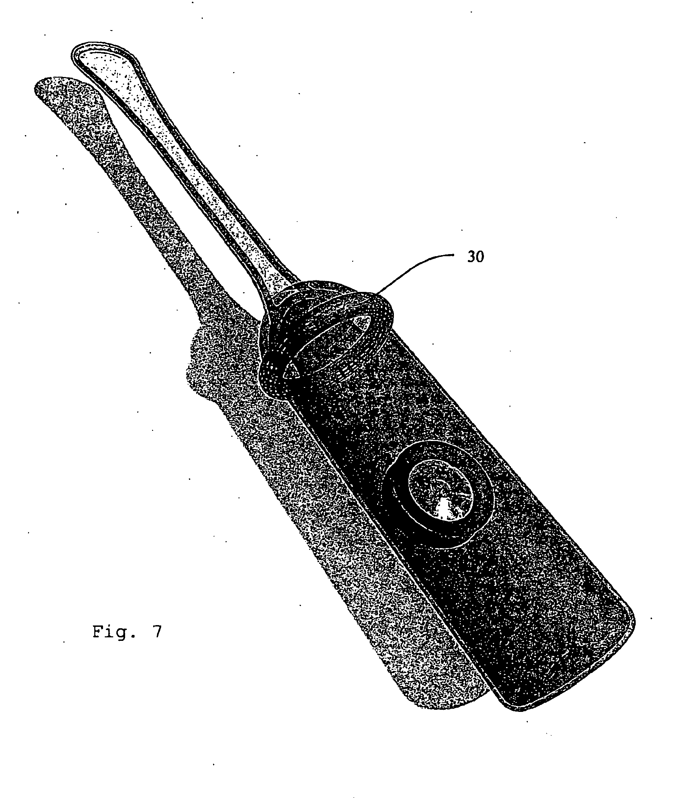

On a représenté aux figures 6 et 7, un autre mode de réalisation du dispositif à la figure 1.

Dans cette variante, l'ensemble formé par le moyen de retrait et le moyen échangeur de chaleur est configuré d'une manière différente.

Ainsi, on remarque à la figure 6 une proéminence 25 circulaire correspondant à une extension de la paroi transversale 3 d'extrémité inférieure, cette proéminence étant surmontée d'une protubérance 26 qui se prolonge en une languette 27 de retrait.

Dans cette protubérance et cette languette, une chambre 28 est ménagée qui prend une forme globalement tubulaire. Comme on l'observe, cette chambre court le long du pourtour intérieur de la languette 27 et épouse sensiblement le contour intérieur de la protubérance 26.

D'autre part, une gorge radiale 29 dans la protubérance loge un anneau torique 30. Cet anneau creux est constitué d'une enveloppe visible à la figure 7, munie d'une multitude de petits orifices ou pores capables de permettre une diffusion lente vers l'extérieur d'un contenant situé à l'intérieur de l'anneau.

### EXEMPLE 3

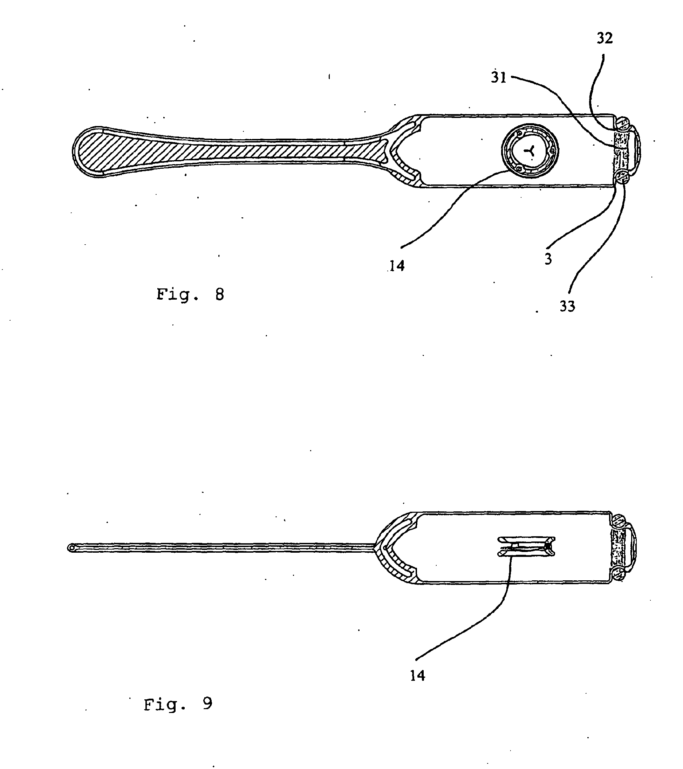

On a représenté aux figures 8 et 9, un autre mode de réalisation du dispositif aux figures 6 et 7.

Dans cette variété, l'ensemble formé par l'anneau torique est positionné de manière différente.

Ainsi, on remarque à la figure 8 une proéminence 31 circulaire correspondant à une extension de la paroi transversale 3 d'extrémité supérieure. D'autre part, une gorge radiale 32 dans cette protubérance loge un anneau torique 33 analogue à l'anneau 30 c'est-à-dire creux et constitué d'une enveloppe munie de pores utiles pour une diffusion lente vers l'extérieure d'un contenant situé à l'intérieur de l'anneau, par exemple une composition contenant des prostaglandines ou leurs dérivés.

Les figures 8 et 9 montrent également le déclencheur 14 disposé à l'intérieur du corps creux rempli d'une solution saline recristallisable, c'est-à-dire une solution aqueuse d'acétate de sodium dans de l'éthylèneglycol contenant de l'aniline.

Le dispositif selon l'invention s'est révélé apte à auto-produire, *in situ,* c'est-à-dire dans la cavité vaginale et jusqu'au niveau du cervix, une quantité de chaleur capable d'élever la température intravaginale jusqu'à atteindre au moins 55°C, selon un accroissement physiologiquement compatible et durant une période de temps suffisante pour obtenir les effets résultants désirés.

D'autre part, l'apport de chaleur suffisant, pour initier notamment un effet contraceptif, peut être obtenu en moins de 5 minutes après introduction vaginale du dispositif selon l'invention dont la solution saline a commencé à cristalliser de manière exothermique.

Grâce à sa capacité de provoquer une hyperthermie intravaginale, le dispositif selon l'invention permet également la production d'autres effets physiologiques bénéfiques notamment la production de protéines de choc thermique (HSPs) ou de protéines de stress thermique et l'acquisition d'un état dit de « thermo-tolérance », protéines dont l'intérêt est explicité en détail précédemment. De tels événements commencent généralement à se produire aux environs de 43°C.

A titre d'exemple, on a enregistré qu'une hyperthermie locale modérée (environ 42°C ou 43°C) durant 15 minutes provoque notamment un accroissement de protéines de choc thermique dont la synthèse cellulaire se poursuit pendant 1 à 2 heures après ce choc thermique physiologique.

D'autre part, un intérêt incontestable du dispositif selon l'invention réside dans la présence d'un échangeur de chaleur qui participe à une uniformisation de l'élévation de la température dans la totalité de la cavité vaginale. Au contraire des dispositifs antérieurs, les effets recherchés, décrits précédemment, peuvent être initiés dès l'entrée vaginale et même au niveau des organes génitaux externes grâce au moyen de retrait lorsqu'il contient un fluide caloporteur.

Il est primordial, en effet, lorsqu'il s'agit par exemple de détruire des spermatozoïdes de manière à initier un effet contraceptif ou d'enrayer la propagation d'un virus tel que le VIH de pouvoir s'assurer qu'aucunes traces de ceux-ci ne subsistent même à l'intérieur du vagin lorsque le dispositif selon l'invention sera extrait de celui-ci.

Au surplus, le dispositif selon l'invention s'est révélé être excellent support pour véhiculer, par l'intermédiaire de l'enveloppe de son corps creux ou des parois transversales de celui-ci, différents types d'agents biocompatibles doués de propriétés médicamenteuses ou non. A ce titre, le dispositif selon l'invention présente un avantage indéniable en ce qu'il permet la pose sur le corps creux d'une gaine support d'agents biocompatibles médicamenteux ou non, cette gaine étant amovible et interchangeable avec des gaines supports analogues.

## Revendications

1. Dispositif capable d'élever la température d'une cavité corporelle, du genre comprenant un corps creux (1) de forme oblongue, pour insertion dans la cavité corporelle, comportant une paroi (2) latérale, une paroi (3) transversale d'extrémité supérieure et une paroi (4) transversale d'extrémité inférieure, ce corps creux étant muni intérieurement d'une source de chaleur et extérieurement d'un moyen (13, 27) non amovible pour le retrait dudit corps creux de la cavité corporelle
**caractérisé en ce que :**
• la paroi transversale d'extrémité inférieure du corps creux est reliée au moyen de retrait par l'intermédiaire d'un moyen (6, 8, 10) apte à échanger de la chaleur entre d'une part ledit corps creux et d'autre part soit ledit moyen de retrait, soit au moins un anneau (12, 30) enserrant ledit moyen échangeur de chaleur, soit ledit moyen de retrait et au moins un anneau enserrant ledit moyen échangeur de chaleur et la paroi transversale d'extrémité supérieure du corps creux comporte éventuellement un moyen (31) apte à échanger de la chaleur entre ledit corps creux et au moins un anneau (33) enserrant ledit moyen échangeur de chaleur,
ou
• la paroi transversale d'extrémité inférieure du corps creux est reliée au moyen de retrait et la paroi transversale d'extrémité supérieure du corps creux comporte un moyen (31) apte à échanger de la chaleur entre ledit corps creux et au moins un anneau (33) enserrant ledit moyen échangeur de chaleur,
en sorte que la source de chaleur génère une quantité de chaleur capable d'augmenter la température de la cavité corporelle totale jusqu'à et y compris son entrée.

2. Dispositif selon la revendication 1, **caractérisée en ce que** le corps creux est relié au moyen de retrait par l'intermédiaire d'un moyen apte à échanger de la chaleur entre ledit corps creux et ledit moyen de retrait.

3. Dispositif selon la revendication 1 ou 2, **caractérisé en ce qu'**un anneau enserre le moyen échangeur de chaleur au niveau de la paroi transversale d'extrémité inférieure.

4. Dispositif selon une des revendications 1 à 3, **caractérisé en ce que** la cavité corporelle est la cavité rectale

5. Dispositif selon une des revendications 1 à 3, **caractérisé en ce que** la cavité corporelle est la cavité vaginale.

6. Dispositif selon une des revendications 1, 2, 3 et 5, **caractérisé en ce que** la source de chaleur génère une quantité de chaleur intravaginale capable :
• d'empêcher la migration des spermatozoïdes dans le cervix et les zones supérieures de fertilisation ainsi que l'acquisition d'une maturité nécessaire à la fécondation de façon à produire un effet contraceptif,
• après un coït et après fécondation, de créer des conditions défavorables au maintien de l'ovule fécondé dans l'utérus et ainsi de produire un effet abortif,
• avant coït, capable de perturber les facteurs qui déterminent la maturité des spermatozoïdes et de défavoriser l'implantation de l'ovule dans la paroi déciduale et, en conséquence, de provoquer son expulsion de l'utérus créant ainsi des conditions contraceptives,
• de déclencher et/ou stimuler le système immunitaire génital féminin.

7. Dispositif selon une des revendications 1 à 6, **caractérisé en ce que** le corps creux comprend une enveloppe réalisée en un matériau de structure spongieuse ou poreuse, apte à être recouvert et/ou imprégné d'agents biocompatibles, médicamenteux ou non.

8. Dispositif selon une des revendications 1 à 6, **caractérisé en ce que** le corps creux est entouré d'une gaine amovible réalisée en un matériau apte à être recouvert et/ou imprégné d'agents biocompatibles, médicamenteux ou non.

9. Dispositif selon une des revendications 1 à 8, **caractérisé en ce que** le moyen de retrait correspond à un cordon (13) de retrait formé d'un brin creux ou non en forme d'anneau ou à une languette (27) de retrait comportant éventuellement un bourrelet périphérique creux ou non, ce cordon de retrait ou cette languette de retrait étant éventuellement recouvert et/ou imprégné d'un ou plusieurs agents biocompatibles médicamenteux ou non choisis parmi des agents spermiostatiques, spermicides, anti-infectieux, virucides et bactéricides.

10. Dispositif selon la revendication 9, **caractérisé en ce que** le brin creux ou la languette de retrait comportant un bourrelet périphérique creux renferme un ou plusieurs agents calorifiques sous forme solide, liquide ou gazeuse sélectionnés parmi des matières capables d'assurer une bonne conduction et/ou convection de la chaleur recueillie à partir du moyen échangeur de chaleur.

11. Dispositif selon une des revendications 1 à 10, **caractérisé en ce que** le moyen apte à échanger de la chaleur comprend une proéminence inférieure (6, 25) provenant de la paroi transversale inférieure surmontée d'une protubérance (8) provenant de cette proéminence ou rapportée sur celle-ci, protubérance dans laquelle le moyen de retrait peut coulisser librement ou être partiellement intégré.

12. Dispositif selon la revendication 11, **caractérisé en ce que** la protubérance, dans laquelle le moyen de retrait peut coulisser librement, est surmontée d'une coiffe (10) amovible.

13. Dispositif selon la revendication 11, **caractérisé en ce qu**'une portion maximale de la surface du moyen de retrait, partiellement intégré dans la protubérance, est en contact avec une portion maximale de cette protubérance elle-même en contact avec une portion maximale de la proéminence inférieure.

14. Dispositif selon une des revendications 1 à 13, **caractérisé en ce que** le moyen apte à échanger de la chaleur comprend une proéminence supérieure (31) provenant de la paroi transversale supérieure ou rapportée sur celle-ci.

15. Dispositif selon une des revendications 1 à 14, **caractérisé en ce que** l'anneau est configuré de manière à assurer un drainage des fluides vaginaux sans blocage de ceux-ci le long de la muqueuse vaginale.

16. Dispositif selon une des revendications 1 à 15, **caractérisé en ce qu'**un anneau circulaire (12, 30, 33) enserre la proéminence inférieure ou la proéminence supérieure ou deux anneaux circulaires enserrent, l'un la proéminence inférieure, l'autre la proéminence supérieure, ces anneaux contenant et/ou étant recouverts et/ou étant imprégnés d'agents biocompatibles médicamenteux ou non.

17. Dispositif selon la revendication 16, **caractérisé en ce que** les agents biocompatibles médicamenteux ou non sont choisis parmi des agents spermiostatiques, spermicides, anti-infectieux, virucides, bactéricides, des prostaglandines naturelles et des dérivés de prostaglandines.

18. Dispositif selon une des revendications 1 à 17, **caractérisé en ce que** la source de chaleur correspond à un système auto-producteur de chaleur *in situ* comprenant une solution saline sursaturée et surrefroidie et un moyen de déclenchement (14) d'une cristallisation exothermique du ou des sels de la solution.

19. Dispositif selon la revendication 18, **caractérisé en ce que** la solution saline comprend au moins un composé inorganique dans un milieu aqueux contenant un alcool et éventuellement une amine ou des nano particules.

20. Dispositif selon la revendication 18 ou 19, **caractérisé en ce que** le moyen de déclenchement comprend un diaphragme (15) de déclenchement comprenant une portion (16) bombée prenant la forme générale d'un triangle équilatéral à côtés légèrement recourbés vers l'intérieur et à sommets fortement arrondis.

21. Dispositif selon la revendication 20, **caractérisé en ce que** l'épaisseur du diaphragme varie depuis son centre jusqu'à son pourtour extérieur en fonction des différentes zones de tension créées lors d'une pression exercée axialement sur ce diaphragme pour en provoquer sa déformation.

22. Dispositif selon la revendication 20 ou 21, **caractérisé en ce que** la portion bombée du diaphragme comporte trois fentes (17), d'égale longueur, qui s'étendent symétriquement à partir de son centre et qui se terminent chacune par un orifice (18), ces orifices étant semblables.

23. Dispositif selon la revendication 22, **caractérisé en ce que** les bords des fentes sont arrondis ou biseautés de façon à éviter tout contact entre eux que le diaphragme soit en position de repos ou en position de déformation.

24. Dispositif selon la revendication 22 ou 23, **caractérisé en ce que** les fentes sont configurées de façon à passer, lors d'une déformation du diaphragme sous l'effet d'une pression, par une position de rapprochement maximal capable de créer une compression de la solution saline entre lesdites fentes avec un dégagement suffisant de chaleur pour initier une cristallisation exothermique du ou des sels de la solution.

25. Dispositif selon une des revendications 20 à 24, **caractérisé en ce que** la portion bombée du diaphragme comprend trois rainures (20) semblables situées entre les orifices et sur une circonférence dont le centre équivaut à celui de ladite portion bombée.

26. Dispositif selon une des revendications 20 à 25, **caractérisé en ce que** le diaphragme comprend, à l'extérieur de la portion bombée, un bourrelet (21) non périphérique qui s'étend transversalement à la fois du côté concave et du côté convexe de ce diaphragme.

27. Dispositif selon une des revendications 20 à 26, **caractérisé en ce que** le diaphragme est fabriqué, à partir d'acier inoxydable, éventuellement combiné à un alliage Ti/Ni, dont la structure hétérogène est constituée de cristaux extrêmement fins.

28. Dispositif selon la revendication 18 ou 19, **caractérisé en ce que** le moyen de déclenchement comprend un diaphragme de déclenchement selon une des revendications 20 à 27, au moins trois pieds supports (19) ainsi que deux éléments (23, 24) de guidage et/ou de protection, l'un et l'autre solidarisées au diaphragme de déclenchement.

## Claims

1. Device capable of increasing the temperature of a body cavity of the type comprising an oblong shaped hollow body (1) for insertion into the body cavity, including a sidewall (2), an upper end cross wall (3) and a lower end cross wall (4), this hollow body being provided with a heat source on the inside and a non removable means (13, 27) for the removal of said hollow body from the vaginal or rectal cavity, whereby:
□ the lower end cross wall of the hollow body is connected to the removal means by a means (6, 8, 10) capable of exchanging heat between firstly a part of said hollow body and secondly either said removal means or at least one ring (12, 30) clamping said heat exchanger means, or said removal means and at least one ring clamping said heat exchanging means, and the upper end cross wall of the hollow body optionally includes a means (31) capable of exchanging heat between said hollow body and at least one ring (33) clamping said heat exchanging means,
or
□ the lower end cross wall of the hollow body is connected to the removal means and the upper end cross wall of the hollow body comprises a means (31) capable of exchanging heat between said hollow body and at least one ring (33) clamping said heat exchanging means,
such that the heat source generates a quantity of heat capable of increasing the temperature of the total body cavity up to and including its entrance.

2. A device according to claim 1, whereby the hollow body is connected to the removal means by a means capable of exchanging heat between said hollow body and said removal means.

3. A device according to claim 1 or 2, whereby the exchanging means in the lower cross wall is clamped with a ring.

4. A device according to one of claims 1 to 3, whereby the body cavity is the rectal cavity.

5. A device according to one of claims 1 to 3, whereby the body cavity is the vaginal cavity.

6. A device according to one of claims 1, 2, 3 and 5, whereby the heat source generates an amount of intravaginal heat capable of:
• preventing migration of spermatozoids in the cervix and the upper zones of fertilisation and acquirement of the necessary maturity for fertilisation, so as to produce a contraceptive effect,
• creating, after coitus and after fertilisation, a lack of keeping of the fertilised ovule in the uterus, so producing an abortive effect,
• perturbing, before coitus, the factors that determine the maturity of spermatozoids and discouraging implantation of the zygote in the decidua wall, thus causing its expulsion from the uterus and so creating contraceptive conditions,
• triggering and/or stimulating the female genital immune system.

7. A device according to one of claims 1 to 6, whereby the hollow body comprises an envelope made of a material with a spongy or porous structure, that can be coated and/or impregnated with biocompatible agents, medicated or non-medicated.

8. A device according to one of claims 1 to 6, whereby the hollow body is surrounded by a removable sheath made of a material that can be coated and/or impregnated with bio-compatible agents, medicated or non-medicated.

9. A device according to one of claims 1 to 8, whereby the removal means corresponds to a removal cord (13) formed from a hollow or solid strand in the form of a ring or a removal tab (27) optionally including a hollow or solid peripheral rim, this removal cord or removal tab being optionally coated and/or impregnated with one or several bio-compatible agents, medicated or non-medicated, chosen from among spermiostatic, spermicide, anti-infectious agents, virucides and bactericides.

10. A device according to claim 9, whereby the hollow peripheral rim or the removal tab including a hollow peripheral rim encloses one or several heat transfer agents in solid, liquid or gas form selected from among materials capable of good conduction and/or convection of heat collected from the heat exchanger.

11. A device according to one of claims 1 to 10, whereby the means capable of exchanging heat comprises a lower prominence (6, 25) originating from the lower cross wall terminating in a protuberance (8) originating from this prominence or added onto it, protuberance in which the removal means can slide freely or be partially integrated.

12. A device according to claim 11, whereby the protuberance in which the removal means can slide freely is covered by a removable cap (10).

13. A device according to claim 11, whereby a maximum portion of the surface of the removal means, partially integrated into the protuberance, is in contact with a maximum portion of this protuberance itself in contact with a maximum portion of the lower prominence.

14. A device according to one of claims 1 to 13, whereby the means capable of exchanging heat comprises an upper prominence (31) originating from the upper cross wall or added onto it.

15. A device according to one of claims 1 to 14, whereby the ring is configured so that it does not prevent flow of vaginal fluids without any blockage of them along the vaginal mucosa.

16. A device according to one of claims 1 to 15, whereby the lower prominence or the upper prominence is clamped by a circular ring (12,30,33) or the lower prominence and the upper prominence are each clamped by a circular ring, these rings containing and/or being coated and/or being impregnated with biocompatible agents, medicated or non-medicated.

17. A device according to claim 16, whereby the biocompatible agents, medicated or non-medicated, are chosen among spermiostatic, spermicide, anti-infectious agents, virucides, bactericides, natural prostaglandins or prostaglandin derivatives.

18. A device according to one of claims 1 to 17, whereby the heat source corresponds to an in situ heat generating system comprising a supersaturated and super-cooled saline solution and a means (14) of triggering exothermic crystallisation of the salt(s) in the solution.

19. A device according to claim 18, whereby the saline solution comprises at least one inorganic compound in an aqueous medium containing an alcohol and optionally an amine or nano particles.

20. A device according to claim 18 or 19, whereby the trigger means (14) comprises a trigger diaphragm (15) which comprises a curved portion (16) in the general shape of an equilateral triangle with sides curved slightly inwards and with rounded vertices.

21. A device according to claim 20, whereby the thickness of the diaphragm varies from its centre towards its outside periphery as a function of the different tension zones thus created during when axial pressure is applied on this diaphragm to cause its deformation.

22. A device according to claim 20 or 21, whereby the curved portion of the diaphragm comprising three slits (17) of equal length that extend symmetrically from its centre, each terminating in an orifice (18), these orifices being similar.

23. A device according to claim 22, whereby the edges of the slits are rounded or bevelled to prevent any contact between them, the diaphragm being in a rest position or in a deformation position.

24. A device according to claim 22 or 23, whereby the slits are configured so that during deformation of the diaphragm under the effect of pressure, they change from a closest together position causing compressing of the saline solution between said slots with sufficient release of heat to initialise exothermal crystallisation of the salt(s) in the solution.

25. A device according to one of claims 20 to 24, whereby the curved portion of the diaphragm comprises three similar grooves (20) located between the orifices and on a circumference with the same centre as said curved portion.

26. A device according to one of claims 20 to 25, whereby the trigger diaphragm comprises a non-peripheral rim (21) outside the curved portion, extending transversely both to the concave side and the convex side of the diaphragm.

27. A device according to one of claims 20 to 26, whereby the diaphragm is made from stainless steel, possibly combined with a Ni/Ti alloy, the heterogeneous structure of which being composed of extremely fine crystals.

28. A device according to claim 18 or 19, whereby the trigger means comprises a trigger diaphragm according to one of claims 20 to 27, at least three support stands (19) and two guide and/or protection elements (23,24), both being fixed to the trigger diaphragm.

## Patentansprüche

1. Vorrichtung, die dazu in der Lage ist, die Temperatur einer Körperhöhle der Art zu erhöhen, die einen hohlen Körper (1) länglicher Form umfasst, um ihn in die Körperhöhle einzuführen, umfassend eine seitliche Wand (2), eine Querwand (3) am oberen Ende und eine Querwand (4) am unteren Ende, wobei der hohle Körper innen mit einer Wärmequelle und außen mit einem nicht abnehmbaren Mittel (13, 27) für den Rückzug des hohlen Körpers aus der Körperhöhle ausgestattet ist, **dadurch gekennzeichnet, dass:**
- die Querwand am unteren Ende des hohlen Körpers mit dem Rückzugsmittel mithilfe eines Mittels (6, 8, 10) verbunden ist, das geeignet ist, Wärme zwischen einerseits dem hohlen Körper und andererseits entweder dem Rückzugsmittel oder mindestens einem Ring (12, 30), der das Wärmeaustauschmittel umschließt, oder dem Rückzugsmittel und mindestens einem Ring, der des Wärmeaustauschmittel umschließt, auszutauschen, und die Querwand des oberen Endes des hohlen Körpers umfasst eventuell ein Mittel (31), das geeignet ist, Wärme zwischen dem hohlen Körper und mindestes einem Ring (33) auszutauschen, der das Wärmeaustauschmittel umschließt,
oder
die Querwand am unteren Ende des hohlen Köpers mit dem Rückzugsmittel verbunden ist, und die Querwand am oberen Ende des hohlen Körpers ein Mittel (31) umfasst, das geeignet ist, Wärme zwischen dem hohlen Körper und mindestens einem Ring (33), der das Wärmeaustauschmittel einschließt, auszutauschen,
so dass die Wärmequelle eine ausreichende Menge Wärme erzeugt, die dazu in der Lage ist, die Temperatur der gesamten Körperhöhle bis und darin eingeschlossen ihren Eingang zu erhöhen.

2. Vorrichtung nach Anspruch 1, **dadurch gekennzeichnet, dass** der hohle Körper mit dem Rückzugsmittel mithilfe eines Mittels verbunden ist, das geeignet ist, Wärme zwischen dem hohlen Körper und dem Rückzugsmittel auszutauschen.

3. Vorrichtung nach Anspruch 1 oder 2, **dadurch gekennzeichnet, dass** ein Ring das Wärmeaustauschmittel auf der Ebene der Querwand am unteren Ende umschließt.

4. Vorrichtung nach einem der Ansprüche 1 bis 3, **dadurch gekennzeichnet, dass** die Körperhöhle die Rektumhöhle ist.

5. Vorrichtung nach einem der Ansprüche 1 bis 3, **dadurch gekennzeichnet, dass** die Körperhöhle die Scheidenhöhle ist.

6. Vorrichtung nach einem der Ansprüche 1, 2, 3 und 5, **dadurch gekennzeichnet, dass** die Wärmequelle eine Menge an intravaginaler Wärme erzeugt, die dazu in der Lage ist:
- die Migration der Spermatozoide in den Hals und die oberen Fertilisierungsbereiche sowie das Erreichen einer Reife, die für die Befruchtung erforderlich ist, zu verhindern, um eine empfängnisverhütende Wirkung zu erzeugen,
- nach einem Koitus und nach der Befruchtung ungünstige Bedingungen für die Rückhaltung der befruchteten Eizelle in der Gebärmutter zu schaffen und so eine Abtreibungswirkung zu erzeugen,
- vor dem Koitus die Faktoren zu stören, die die Reife der Spermatozoide bestimmen und die Einpflanzung der Eizelle in der Dezidualwand ungünstig zu beeinflussen und, folglich, ihre Ausstoßung aus der Gebärmutter hervorzurufen und somit empfängnisverhütende Bedingungen zu schaffen,
- das weibliche genitale Immunsystem auszulösen und/oder zu stimulieren.

7. Vorrichtung nach einem der Ansprüche 1 bis 6, **dadurch gekennzeichnet, dass** der hohle Körper eine Ummantelung umfasst, die aus einem Material mit schwammartiger oder poröser Struktur hergestellt ist, das geeignet ist, mit biokompatiblen Wirkstoffen medikamentöser Art oder nicht bedeckt oder getränkt zu werden.

8. Vorrichtung nach einem der Ansprüche 1 bis 6, **dadurch gekennzeichnet, dass** der hohle Körper von einer abnehmbaren Hülle umgeben ist, die aus einem Material hergestellt ist, das geeignet ist, mit biokompatiblen Wirkstoffen medikamentöser Art oder nicht bedeckt oder getränkt zu werden.

9. Vorrichtung nach einem der Ansprüche 1 bis 8, **dadurch gekennzeichnet, dass** das Rückzugsmittel einer Rückzugsschnur (13), gebildet aus einem hohlen oder nicht hohlen Strang in Form eines Ringes oder einer Rückzugslasche (27), entspricht, umfassend eventuell einen hohlen oder nicht hohlen Umfangs-Dichtungsstreifen, wobei diese Rückzugsschnur oder diese Rückzugslasche eventuell mit einem oder mit mehreren biokompatiblen Wirkstoffen medikamentöser Art oder nicht abgedeckt oder getränkt ist, die unter spermiostatischen, spermatoziden, infektionsverhütenden, viriziden und bakteriziden Wirkstoffen ausgewählt werden.

10. Vorrichtung nach Anspruch 9, **dadurch gekennzeichnet, dass** der hohle Strang oder die Rückzugslasche, umfassend einen hohlen Umfangs-Dichtungsstreifen, ein oder mehrere Wärmemittel in festem, flüssigem oder gasförmigem Zustand einschließt, die unter Materialien ausgewählt wurden, die dazu in der Lage sind, eine gute Leitung und/oder Konvektion der Wärme, die mithilfe des Wärmetauschmittels gesammelt wurde, sicherzustellen.

11. Vorrichtung nach einem der Ansprüche 1 bis 10, **dadurch gekennzeichnet, dass** das Mittel, das geeignet ist, Wärme auszutauschen, einen unteren Vorsprung (6, 25) umfasst, der sich aus der unteren Querwand erstreckt, über der eine Noppe (8) liegt, die sich aus diesem Vorsprung erstreckt oder auf diesen bezogen ist, wobei in der Noppe das Rückzugsmittel frei gleiten oder teilweise integriert sein kenn.

12. Vorrichtung nach Anspruch 11, **dadurch gekennzeichnet, dass** die Noppe, in der das Rückzugsmittel frei gleiten kann, von einer abnehmbaren Haube (10) überlagert ist.

13. Vorrichtung nach Anspruch 11, **dadurch gekennzeichnet, dass** ein maximaler Teil der Oberfläche des Rückzugsmittels, das teilweise in die Noppe integriert ist, in Kontakt mit einem maximalen Teil dieser Noppe ist, die ihrerseits in Kontakt mit einem maximalen Teil des unteren Vorsprungs steht.

14. Vorrichtung nach einem der Ansprüche 1 bis 13, **dadurch gekennzeichnet, dass** das Mittel, das geeignet ist, Wärme auszutauschen, einen oberen Vorsprung (31) umfasst, der sich aus der oberen Querwand erstreckt oder auf diese bezogen ist.

15. Vorrichtung nach einem der Ansprüche 1 bis 14, **dadurch gekennzeichnet, dass** der Ring so gestaltet ist, dass er eine Drainage der Vaginalflüssigkeiten sicherstellt, ohne diese entlang der vaginalen Schleimhaut zu blockieren.

16. Vorrichtung nach einem der Ansprüche 1 bis 15, **dadurch gekennzeichnet, dass** ein Kreisring (12, 30, 33) den unteren Vorsprung oder den oberen Vorsprung umfasst, oder dass von zwei Kreisringen der eine den unteren Vorsprung und der andere den oberen Vorsprung umfasst, wobei diese Ringe biokompatible Wirkstoffe medikamentöser Art oder nicht enthalten und/oder davon bedeckt und/oder getränkt sind.

17. Vorrichtung nach Anspruch 16, **dadurch gekennzeichnet, dass** die biokompatiblen Wirkstoffe medikamentöser Art oder nicht unter spermiostatischen, spermatoziden, infektionsverhütenden, viriziden und bakteriziden Wirkstoffen, natürlichen Prostaglandinen und Derivaten von Prostaglandinen ausgewählt werden.

18. Vorrichtung nach einem der Ansprüche 1 bis 17, **dadurch gekennzeichnet, dass** die Wärmequelle einem Wärmeselbsterzeugungssystem in situ entspricht, umfassend eine übersättigte und unterkühlte Salzlösung und ein Auslösungsmittel (14) einer exothermischen Kristallisierung des oder der Salze der Lösung.

19. Vorrichtung nach Anspruch 18, **dadurch gekennzeichnet, dass** die Salzlösung mindestens eine anorganische Verbindung in einem wässrigen Medium umfasst, das ein Alkohol und eventuell ein Amin oder Nanopartikel enthält.

20. Vorrichtung nach Anspruch 18 oder 19, **dadurch gekennzeichnet, dass** das Auslösungsmittel eine Auslösungsmembran (15) aufweist, umfassend einen gewölbten Abschnitt (16), der die allgemeine Form eines gleichseitigen Dreiecks mit leicht nach innen gekrümmten Seiten und mit stark abgerundeten Scheiteln aufweist.

21. Vorrichtung nach Anspruch 20, **dadurch gekennzeichnet, dass** die Dicke der Membran von ihrer Mitte bis zu ihrem äußeren Umfang je nach den verschiedenen Spannungsbereichen variiert, die durch einen Druck, der axial auf diese Membran ausgeübt wird, um ihre Verformung hervorzurufen, erzeugt werden.

22. Vorrichtung nach Anspruch 20 oder 21, **dadurch gekennzeichnet, dass** der gewölbte Abschnitt der Membran drei Schlitze (17) mit gleicher Länge aufweist, die sich symmetrisch aus ihrer Mitte erstrecken und jeweils mit einer Öffnung (18) enden, wobei diese Öffnungen ähnlich sind.

23. Vorrichtung nach Anspruch 22, **dadurch gekennzeichnet, dass** die Ränder der Schlitze abgerundet oder abgeschrägt sind, um jeden Kontakt zwischen ihnen zu vermeiden, unabhängig davon, ob sich die Membran in der Ruhe- oder der Verformungsposition befindet.

24. Vorrichtung nach Anspruch 22 oder 23, **dadurch gekennzeichnet, dass** die Schlitze so gestaltet sind, dass sie bei einer Verformung der Membran unter der Einwirkung eines Drucks eine Position der maximalen Annäherung durchlaufen, die dazu in der Lage ist, eine Verdichtung der Salzlösung zwischen den Schlitzen mit einer ausreichenden Wärmeabgabe zu schaffen, um eine exothermische Kristallisierung des oder der Salze der Lösung einzuleiten.

25. Vorrichtung nach einem der Ansprüche 20 bis 24, **dadurch gekennzeichnet, dass** der gewölbte Abschnitt der Membran drei ähnliche Nuten (20) umfasst, die sich zwischen den Öffnungen und auf einem Umfang befinden, dessen Mitte derjenigen des gewölbten Abschnittes entspricht.

26. Vorrichtung nach einem der Ansprüche 20 bis 25, **dadurch gekennzeichnet, dass** die Membran außerhalb des gewölbten Abschnittes einen nicht peripheren Umfangs-Dichtungsstreifen (21) umfasst, der sich quer sowohl zur konkaven als auch zur konvexen Seite dieser Membran erstreckt.

27. Vorrichtung nach einem der Ansprüche 20 bis 26, **dadurch gekennzeichnet, dass** die Membran aus Edelstahl hergestellt ist, eventuell in Verbindung mit einer Ti/Ni-Legierung, dessen heterogene Struktur aus extrem feinen Kristallen gebildet ist.

28. Vorrichtung nach Anspruch 18 oder 19, **dadurch gekennzeichnet, dass** das Auslösungsmittel eine Auslösungsmembran nach einem der Ansprüche 20 bis 27, mindestens drei Stützfüße (19) sowie zwei Elemente (23, 24) zur Führung und/oder zum Schutz umfasst, wobei beide mit der Auslösemembran fest verbunden sind.
